# EUROPEAN PATENT APPLICATION

(11) **EP 4 095 530 A1**
(43) Date of publication of application: **30.11.2022**
(21) Application number: 20915043.2
(22) Date of filing: 16.09.2020
(51) Int. Cl.: G01N 33/50, G01N 35/10

(54) **IN-VITRO DIAGNOSTIC ANALYZER, REAGENT CARD, AND MOUNTING STRUCTURE**

(30) Priority: 23.01.2020 CN 202010076460
(71) Applicant: Guangzhou Wondfo Biotech Co., Ltd., Guangzhou, Guangdong 510663 (CN)
(72) Inventor: HUANG, Yurong, Guangzhou, Guangdong 510663 (CN); WANG, Jihua, Guangzhou, Guangdong 510663 (CN)
(74) Representative: Hannke, Christian
(86) International application number: PCT/CN2020/115468
(87) International publication number: WO 2021/147352

(57) **Abstract**

An *in-vitro* diagnostic analyzer, a reagent card (10), and an installation structure (200) are disclosed. The installation structure (200) includes an installation body (210). The installation body (210) includes an installation hole (212) configured to sleeve a sample tube (70), a hollow needle (220) provided in the installation hole (212), a sealing portion (240) provided in the installation hole (212), and an air inlet channel (230). One end of the hollow needle (220) is capable of being inserted into the sample tube (70). The sealing portion (240) is in sealing fit with an outer wall of the sample tube (70). The air inlet channel (230) includes an air outlet hole (234) and an air inlet hole (232) provided on a surface of the installation body (210). The air outlet hole (234) is configured for communication with the sample tube (70) provided on the installation hole (212). The reagent card (10) is integrated with the installation structure (200), and the *in-vitro* diagnostic analyzer is integrated with the reagent card (10). On-off control of a sample solution is implemented by opening or closing the air inlet hole (232), and the problems that the sample solution leaks and cannot be extracted are solved.

## Description

### CROSS REFERENCE TO RELATED APPLICATION

This application claims priority to Chinese Patent Application No. 202010076460.8 filed on January 23, 2020, which is incorporated herein by reference in its entirety.

### TECHNICAL FIELD

This application relates to the technical field of medical devices, for example, to an *in-vitro* diagnostic analyzer and a reagent card.

### BACKGROUND

Reagent cards (also known as test cards or detection cards) are widely used in the medical industry. The reagent card integrates biochemical detection electrodes. Calibration is conducted by the calibration liquid, and then the sample solution (blood, tissue fluid, etc.) is detected.

The reagent card in the related art usually realizes the on-off control of the sample solution by closing and opening the sample inlet channel through extrusion. However, it has the following problems:
1. There is a hidden danger that the sample solution leaks along the tubing: when the reagent card is not inserted in place or the size of the instrument assembly is deviated, the pressing part is easy to be deviated, such that the sample inlet channel cannot be compressed and closed. In this way, during calibration analysis, the sample solution will accidentally flow into the reagent card, resulting in distortion of the calibration analysis.
2. There is a hidden danger that the sample solution cannot be extracted: when the ambient temperature of the instrument is too low or the negative pressure in the reagent card is too large, the elastic body of the sample inlet channel will rebound poorly or even fail to rebound, such that the sample solution fails to flow into the reagent card.

### SUMMARY

This application provides an *in-vitro* diagnostic analyzer, a reagent card, and an installation structure. The installation structure can install the sample tube, and can realize on-off control of a sample solution, so as to solve the above-mentioned hidden dangers that the sample solution leaks and cannot be extracted. The reagent card is integrated with the above-mentioned installation structure, and adopts a new on-off control scheme for the sample solution, which can solve the above-mentioned hidden dangers that the sample solution leaks and cannot be extracted. When the *in-vitro* diagnostic analyzer is used, the on-off control of the sample solution is reliable, which is beneficial to improve the reliability of detection.

The embodiments of the present disclosure provide an installation structure, including an installation body. The installation body includes an installation hole configured to sleeve a sample tube, a hollow needle provided in the installation hole, a sealing portion provided in the installation hole, and an air inlet channel. One end of the hollow needle is capable of being inserted into the sample tube. The sealing portion is in sealing fit with an outer wall of the sample tube. The air inlet channel includes an air outlet hole and an air inlet hole. The air inlet hole is provided on a surface of the installation body. The air outlet hole is configured for communication with the sample tube provided on the installation hole.

When the above installation structure is used, the sample tube can be inserted into the installation hole. In this process, the hollow needle will be inserted into the sample tube. After the sample tube is installed, the sealing portion will be in sealing fit with the sample tube. At this time, when the air inlet hole is opened, gas (such as air) can enter the sample tube through the air inlet channel, that is, liquid in the sample tube can be sucked away through the hollow needle at this time. When the air inlet hole is closed, gas (such as air) cannot enter the sample tube through the air inlet channel, and the sample tube is sealed by the sealing portion, such that the sample tube cannot communicate with the external gas, making it difficult for the liquid in the sample tube to be sucked out through the hollow needle. The installation structure can realize the installation of the sample tube. During the on-off control of the sample solution, there is no need to deform the sample inlet channel through extrusion, so as to close or rely on the self-reset of the sample inlet channel to open the sample inlet channel. It is only necessary to open or close the air inlet hole to realize the on-off control of the sample solution, which is beneficial to solve the above-mentioned hidden dangers that the sample solution leaks and cannot be extracted.

In some embodiments, the sealing portion may be provided with a sealing hole in sealing fit with a sample outlet end of the sample tube. One end of the hollow needle may be arranged in the sealing hole. The air outlet hole may communicate with the sealing hole.

In some embodiments, an outer edge of the air inlet hole may be provided with a sealing layer.

In some embodiments, the sealing layer may include an elastic layer and an adhesive layer arranged on the elastic layer, and the elastic layer may be arranged facing outward.

The embodiments of the present disclosure further provide a reagent card, including the installation structure in any of the above embodiments, and further including a reagent card body. The reagent card body is fixed to the installation structure. The reagent card body includes a sample inlet channel communicating with a liquid outlet end of the hollow needle, a detection chamber, and an air receiving end. The sample inlet channel and the air receiving end communicate with the detection chamber.

When the above reagent card is used, the sample tube can be inserted into the installation hole in the installation body. In this process, the hollow needle will be inserted into the sample tube. After the sample tube is installed, the sealing portion will be in sealing fit with the sample tube. At this time, when the air inlet hole is opened, gas (such as air) can enter the sample tube through the air inlet channel, that is, the air receiving end is in connection with a negative pressure generator at this time to generate suction, such that liquid in the sample tube can be sucked into the sample inlet channel through the hollow needle, and flow into the detection chamber for detection and analysis. When the air inlet hole is closed, gas (such as air) cannot enter the sample tube through the air inlet channel, and the sample tube is sealed by the sealing portion, such that the sample tube cannot communicate with the external gas, making it difficult for the liquid in the sample tube to be sucked out through the hollow needle. During the on-off control of the sample solution by the reagent card, there is no need to deform the sample inlet channel through extrusion, so as to close or rely on the self-reset of the sample inlet channel to open the sample inlet channel. It is only necessary to open or close the air inlet hole through a telescopic valve to realize the on-off control of the sample solution, which can solve the above-mentioned hidden dangers that the sample solution leaks and cannot be extracted.

In some embodiments, the installation body and the reagent card body may be integrally formed.

In some embodiments, the reagent card body may further include a calibration liquid channel, and the calibration liquid channel may communicate with the detection chamber.

In some embodiments, the reagent card body may further include a waste liquid storage chamber communicating with a liquid outlet end of the detection chamber, and a liquid outlet end of the waste liquid storage chamber may communicate with the air receiving end.

In some embodiments, the reagent card body may further include a first calibration liquid bag and a valve core. The first calibration liquid bag may be fixed on the reagent card body. The first calibration liquid bag may be provided with a matching portion in connection with the calibration liquid channel. The valve core may be arranged in the calibration liquid channel or in the first calibration liquid bag. The valve core may be provided with a spiked portion configured to pierce the matching portion.

The embodiments of the present disclosure further provide an *in-vitro* diagnostic analyzer, including the reagent card in any of the above embodiments, and further including a negative pressure generator, a sample tube, a first retractor, a detection device, and a controller. The negative pressure generator is in connection with the air receiving end. The sample tube is provided with a sample outlet end in clearance fit with the hollow needle. The first retractor is provided with a sealing end configured to seal the air inlet hole. The controller telecommunicates with the negative pressure generator, the first retractor, and the detection device. A detection end of the detection device is arranged in the detection chamber.

When the above *in-vitro* diagnostic analyzer is used, the sample tube can be inserted into the installation hole in the installation body. In this process, the hollow needle will be inserted into the sample tube. After the sample tube is installed, the sealing portion will be in sealing fit with the sample tube. Then the reagent card is installed at a preset position of the *in-vitro* diagnostic analyzer. When the first controller controls the action of the first retractor to make the sealing end leave the air inlet hole, that is, the air inlet hole is opened, gas (such as air) can enter the sample tube through the air inlet channel, that is, the air receiving end is in connection with the negative pressure generator at this time to generate suction, such that liquid in the sample tube can be sucked into the sample inlet channel through the hollow needle, and flow into the detection chamber for detection and analysis. When the controller controls the action of the first retractor to make the sealing end be in sealing fit the air inlet hole, that is, the air inlet hole is closed, gas (such as air) cannot enter the sample tube through the air inlet channel, and the sample tube is sealed by the sealing portion, such that the sample tube cannot communicate with the external gas, making it difficult for the liquid in the sample tube to be sucked out through the hollow needle. When the *in-vitro* diagnostic analyzer is used, the on-off control of the sample solution is reliable, which is beneficial to improve the reliability of detection.

In some embodiments, the sealing end may be provided with a protruding sealing ring, and the sealing ring can be hermetically arranged at an outer edge of the air inlet hole.

In some embodiments, the reagent card body may further include a calibration liquid channel communicating with the detection chamber, a first calibration liquid bag, and a valve core. The first calibration liquid bag may be fixed on the reagent card body. The first calibration liquid bag may be provided with the matching portion in connection with the calibration liquid channel. The valve core may be arranged in the calibration liquid channel or in the first calibration liquid bag. The valve core may be provided with a spiked portion configured to pierce the matching portion. The *in-vitro* diagnostic analyzer may further include a second retractor that telecommunicates with the controller. A telescopic end of the second retractor is capable of pressing the calibration liquid channel or the first calibration liquid bag in a preset direction, such that the spiked portion may pierce the matching portion.

In some embodiments, the *in-vitro* diagnostic analyzer may further include the calibration liquid channel that communicates with the detection chamber, and a second calibration liquid bag. The second calibration liquid bag may communicate with the calibration liquid channel through an on-off valve.

### BRIEF DESCRIPTION OF THE DRAWINGS

FIG. 1 is a schematic structural diagram of an *in-vitro* diagnostic analyzer shown in an embodiment;
FIG. 2 is a schematic structural diagram of an *in-vitro* diagnostic analyzer shown in an embodiment;
FIG. 3 is a schematic structural diagram of a reagent card shown in FIG. 2;
FIG. 4 is a partially enlarged view of A shown in FIG. 3;
FIG. 5 is a schematic diagram of a reagent card shown in FIG. 3 sucking calibration liquid;
FIG. 6 is a schematic diagram after the reagent card shown in FIG. 3 completes calibration analysis;
FIG. 7 is a schematic diagram of a state of an air inlet hole in a reagent card state shown in FIG. 5 or FIG. 6;
FIG. 8 is a schematic diagram of the reagent card shown in FIG. 3 sucking a sample solution;
FIG. 9 is a partially enlarged view of B shown in FIG. 8;
FIG. 10 is a schematic diagram of a state of the air inlet hole in a reagent card state shown in FIG. 8;
FIG. 11 is a schematic structural diagram of the reagent card shown in FIG. 1; and
FIG. 12 is a schematic structural diagram of second calibration liquid and a valve core of the reagent card shown in FIG. 11.

### Reference numerals:

10, reagent card; 100, reagent card body; 110, sample inlet channel; 120, detection chamber; 130, air receiving end; 140, calibration liquid channel; 150, first calibration liquid bag; 160, matching portion; 170, valve core; 172, spiked portion; 180, waste liquid storage chamber; 200, installation structure; 210, installation body; 212, installation hole; 220, hollow needle; 230, air inlet channel; 232, air inlet hole; 234, air outlet hole; 240, sealing portion; 242, sealing hole; 250, sealing layer; 252, elastic layer; 254, adhesive layer; 20, negative pressure generator; 40, first retractor; 42, sealing end; 44, sealing ring; 50, second retractor; 60, controller; 70, sample tube; and 80, second calibration liquid bag.

### DETAILED DESCRIPTION

It should be noted that when a component is "fixed", "provided", "fixed on", or "provided on" another component, the component may be fixed on the other component directly or via an intermediate component. When a component is connected with the other component, the component may be connected with the other component directly or via an intermediate component. Further, when an element is considered to be "connected" with another element, the two can be fixed by detachable connection, or can be fixed by non-detachable connection, such as sleeve connection, snap connection, integral molding fixing, and welding, which can be implemented in the related art, and is not redundant here. When one component and another component are perpendicular or approximately perpendicular to each other, it means that the ideal state of the two is vertical, but due to the influence of manufacturing and assembly, there may be a certain vertical error. The terms "vertical", "horizontal", "left", and "right" and similar expressions used herein are just for illustrative purposes, and do not mean sole implementations.

Unless otherwise defined, all technical and scientific terms used herein have the same meaning as commonly understood by those skilled in the technical field of this application. The terms used herein in the specification of this application are merely for the purpose of describing specific embodiments, and are not intended to limit this application. The term "and/or" used herein includes any and all combinations of one or more of the associated listed items.

The "first" and "second" mentioned in this application do not represent the specific quantity and order, and they are merely used to distinguish the name.

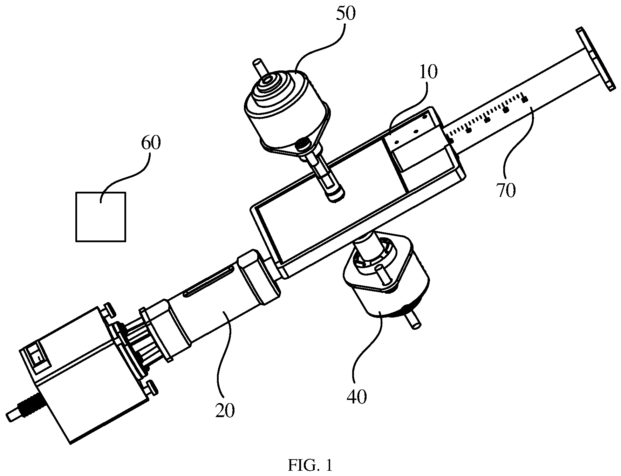

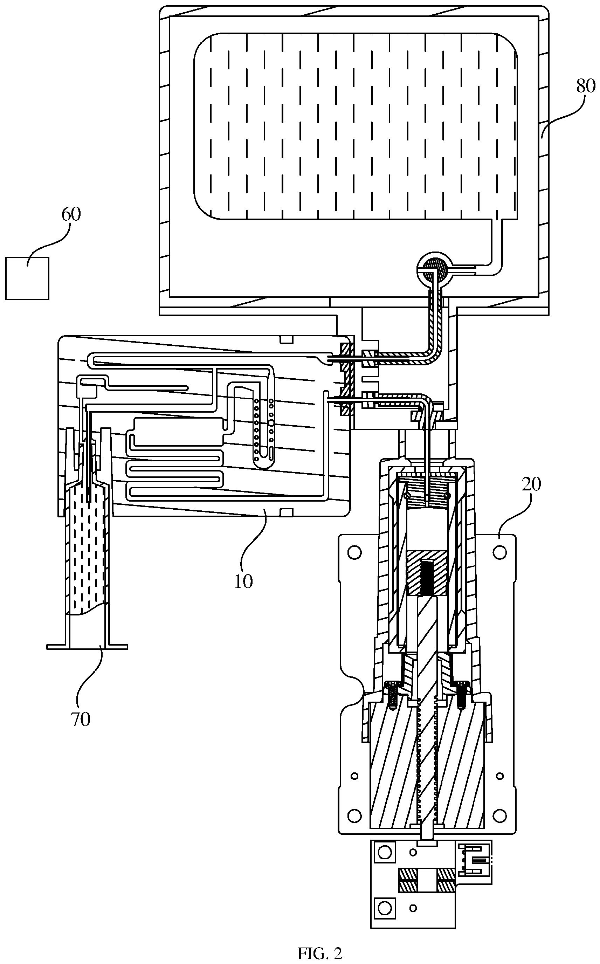

As shown in FIG. 1 and FIG. 2, this application further provides an *in-vitro* diagnostic analyzer, including a reagent card 10, a negative pressure generator, a sample tube 70, a first retractor 40, a detection device, and a controller 60.

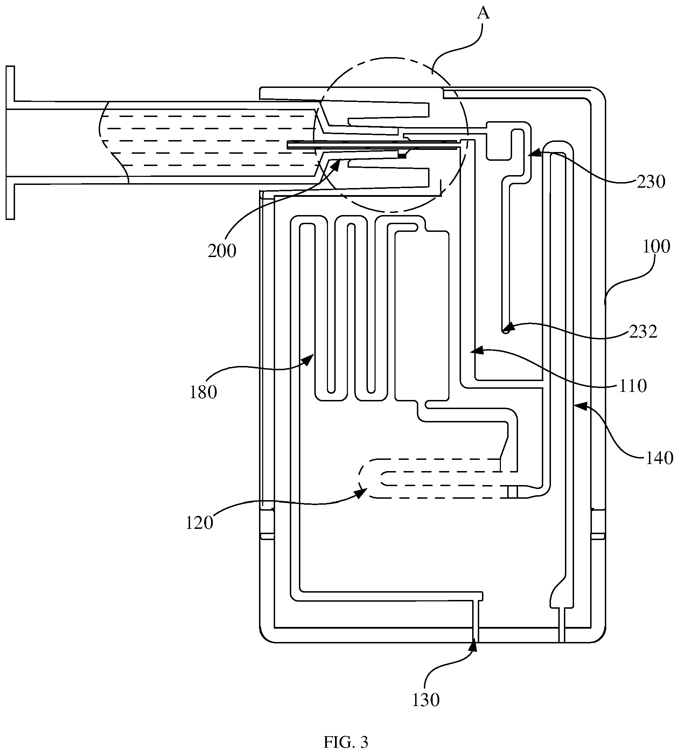

As shown in FIG. 3, FIG. 4, and FIG. 7, the reagent card 10 includes a reagent card body 100 and an installation structure 200 fixed on the reagent card body. The reagent card body 100 includes a sample inlet channel 110, a detection chamber 120, and an air receiving end 130. The sample inlet channel 110 and the air receiving end 130 communicate with the detection chamber 120. The installation structure 200 includes an installation body 210. The an installation body 210 includes an installation hole 212 configured to sleeve the sample tube 70, a hollow needle 220 provided in the installation hole 212, a sealing portion 240 provided in the installation hole 212, and an air inlet channel 230. The hollow needle 220 includes one end capable of being inserted into the sample tube 70, and the other end inserted into the sample inlet channel 110. The sealing portion 240 is in sealing fit with an outer wall of the sample tube 70. The air inlet channel 230 includes an air outlet hole 234 and an air inlet hole 232. The air inlet hole is provided on a surface of the installation body 210. The air outlet hole 234 is configured for communication with the sample tube 70 provided on the installation hole 212.

The negative pressure generator is in connection with the air receiving end 130. The sample tube 70 is provided with a sample outlet end in clearance fit with the hollow needle 220. The first retractor 40 is provided with a sealing end 42 configured to seal the air inlet hole 232. The controller 60 telecommunicates with the negative pressure generator 20, the first retractor 40, and the detection device. A detection end of the detection device is arranged in the detection chamber 120.

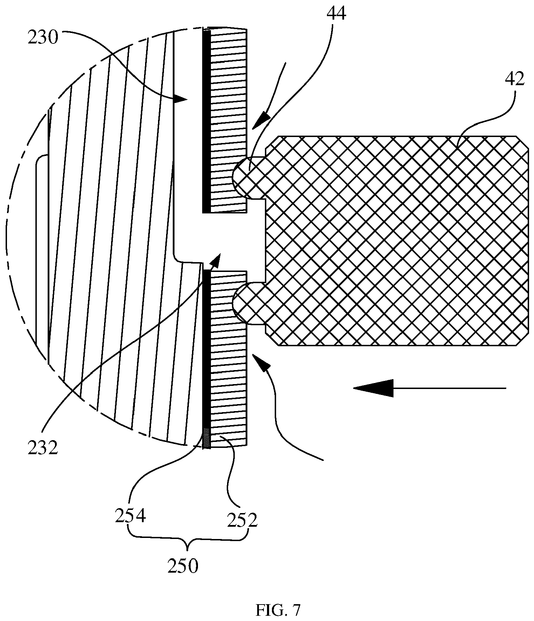

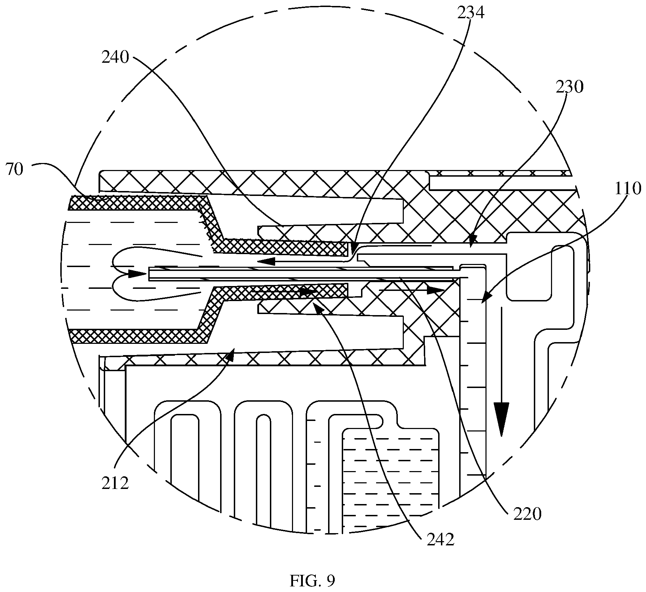

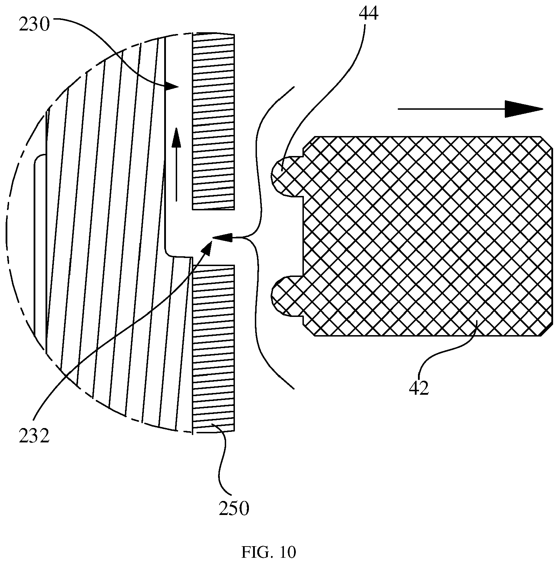

When the above *in-vitro* diagnostic analyzer is used, the sample tube 70 can be inserted into the installation hole 212 in the installation body 210. In this process, the hollow needle 220 will be inserted into the sample tube 70. After the sample tube 70 is installed, the sealing portion 240 will be in sealing fit with the sample tube 70. Then the reagent card 10 is installed at a preset position of the *in-vitro* diagnostic analyzer. When the first controller 60 controls the action of the first retractor 40 to make the sealing end 42 leave the air inlet hole 232, that is, the air inlet hole 232 is opened (as shown in FIG. 9 and FIG. 10), gas (such as air) can enter the sample tube 70 through the air inlet channel 230, that is, if the air receiving end 130 is in connection with the negative pressure generator 20 at this time to generate suction, liquid in the sample tube 70 can be sucked into the sample inlet channel 110 through the hollow needle 220, and flow into the detection chamber 120 for detection and analysis by the detection device. When the controller 60 controls the action of the first retractor 40 to make the sealing end 42 be in sealing fit the air inlet hole 232, that is, the air inlet hole 232 is closed (as shown in FIG. 7), gas (such as air) cannot enter the sample tube 70 through the air inlet channel 230, and the sample tube 70 is sealed by the sealing portion 240, such that the sample tube 70 cannot communicate with the external gas, making it difficult for the liquid in the sample tube 70 to be sucked out through the hollow needle 220. When the *in-vitro* diagnostic analyzer is used, the on-off control of the sample solution is reliable, which is beneficial to improve the reliability of detection.

Optionally, the "negative pressure generator 20" can be any related liquid suction equipment such as a suction pump and a vacuum pump that meets the requirements of this application.

Optionally, the "controller 60" includes, but is not limited to, a programmable controller 60, a motion control card, and a computer.

Optionally, the "sealing portion 240" includes, but is not limited to, structures such as a sealing ring, a sealing sleeve, and a sealing layer, as long as the sample tube 70 can be sealed and fixed in the installation hole 212.

Optionally, the "sample tube 70" includes structures such as a syringe and a test tube.

Optionally, the expression that "one end of the hollow needle 220 is capable of being inserted into the sample tube" can be understood as one end of the hollow needle 220 protruding from the bottom of the installation hole 212 to form an interface.

The "bottom of the installation hole 212" can be understood as the bottom wall corresponding to the sample outlet end of the sample tube 70 after the sample tube 70 is inserted, including but not limited to those shown in FIG. 1, but also other equivalent solutions after deformation.

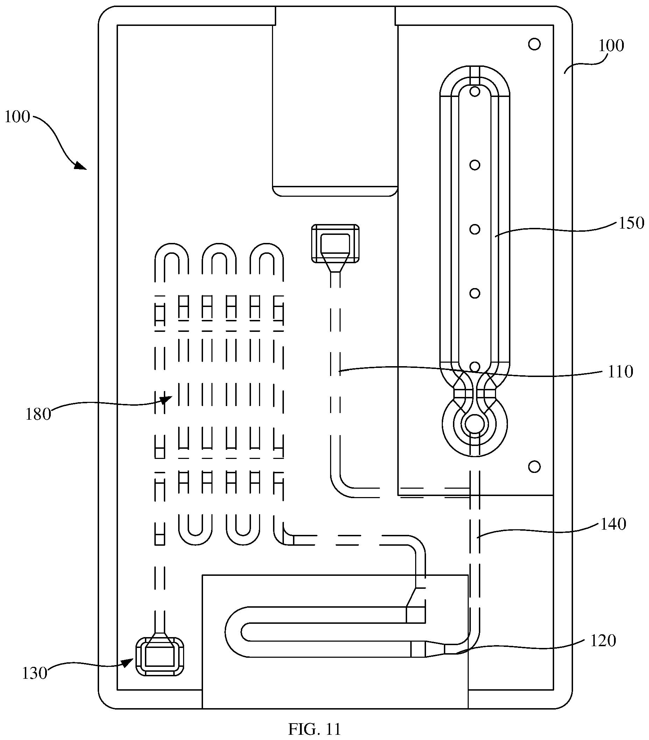

On the basis of the above embodiment, as shown in FIG. 3 or FIG. 11, in an embodiment, the reagent card body 100 further includes a calibration liquid channel 140, and the calibration liquid channel 140 communicates with the detection chamber 120. In this way, the reagent card 10 can introduce the calibration liquid into the detection chamber 120 through the calibration liquid channel 140 for calibration, and detect the sample solution.

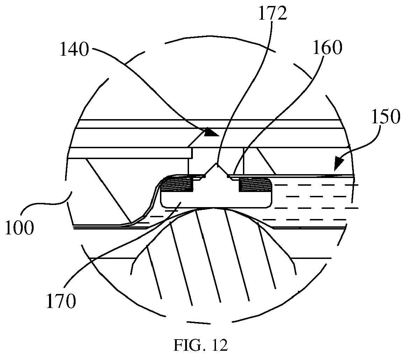

On the basis of the above embodiment, as shown in FIG. 11 to FIG. 12, in an embodiment, the reagent card body 100 further includes a first calibration liquid bag 150 and a valve core 170. The first calibration liquid bag 150 is fixed on the reagent card body 100. The first calibration liquid bag 150 is provided with a matching portion 160 in connection with the calibration liquid channel 140. The valve core 170 is arranged in the calibration liquid channel 140 or in the first calibration liquid bag 150. The valve core 170 is provided with a spiked portion 172 configured to pierce the matching portion 160. The *in-vitro* diagnostic analyzer further includes a second retractor 50 that telecommunicates with the controller 60. A telescopic end of the second retractor 50 is capable of pressing the calibration liquid channel 140 or the first calibration liquid bag 150 in a preset direction, such that the spiked portion 172 pierces the matching portion 160.

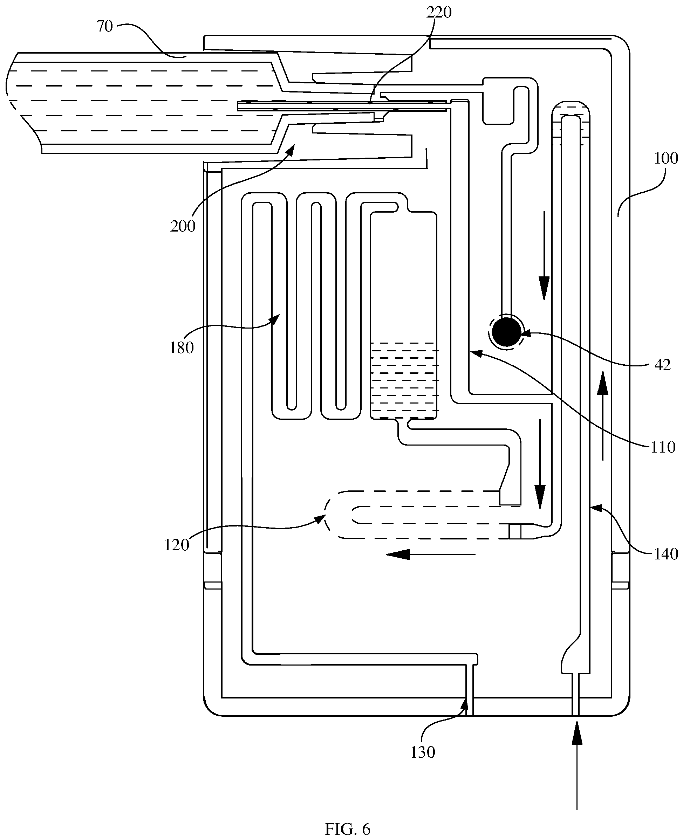

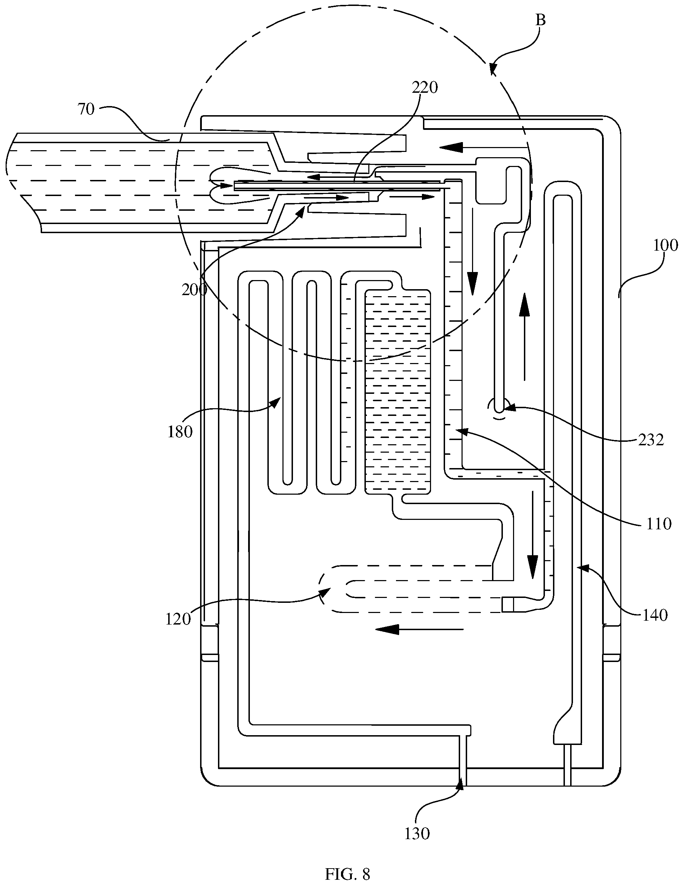

At this time, the calibration liquid can be integrated into the reagent card body 100 . With reference to FIG. 1 and FIG. 5 to FIG. 9, in use, the sample tube 70 can be inserted into the installation hole 212 in the installation body 210. In this process, the hollow needle 220 will be inserted into the sample tube 70. After the sample tube 70 is installed, the sealing portion 240 will be in sealing fit with the sample tube 70. Then the reagent card 10 is installed at a preset position of the *in-vitro* diagnostic analyzer. The controller 60 controls the action of the first retractor 40, such that the sealing end 42 is in sealing fit with the air inlet hole 232, that is, the air inlet hole 232 is closed. Then, the calibration analysis is performed. The controller 60 controls the telescopic end of the second retractor 50 to press the calibration liquid channel 140 or the first calibration liquid bag 150 in a preset direction, driving the spiked portion 172 of the valve core 170 to pierce the matching portion 160, such that the calibration liquid in the first calibration liquid bag 150 flows into the calibration liquid channel 140. The controller 60 then controls the action of the negative pressure generator 20 to send the calibration liquid into the detection chamber 120 for calibration, and uses the detection device to analyze the calibration liquid to complete the calibration analysis. After the calibration analysis is completed, the negative pressure generator 20 continues to operate, such that the calibration liquid is extracted out of the detection chamber 120. At this time, the action of the first retractor 40 is controlled, such that the air inlet hole 232 is opened. At this time, the negative pressure generator 20 continues to operate, and an appropriate amount of sample solution (such as blood) can be sucked into the detection chamber 120. Then the detection device is used to analyze the detection solution to obtain an analysis report of the sample solution. During calibration, a blood gas analyzer can accurately control the extraction volume of the calibration liquid, ensure that the calibration liquid completely covers a detection end of the detection device, accurately complete the calibration analysis, and obtain accurate calibration parameters. In this process, the on-off control of the air inlet hole 232 is reliable, and the sample solution will not interfere with the calibration analysis. During analysis, the sample solution is sucked smoothly, which is beneficial to the smooth progress of the sample solution detection and improves the reliability.

In another embodiment, as shown in FIG. 2, the *in-vitro* diagnostic analyzer further includes a second calibration liquid bag 80, and the second calibration liquid bag 80 communicates with the calibration liquid channel 140 through an on-off valve (not marked). Similarly, at this time, the second calibration liquid bag 80 can be placed outside the reagent card body 100, and abutment can be realized by tubings and on-off valves, such that the calibration liquid can be stored in the *in-vitro* diagnostic analyzer. The on-off valve is used to realize on-off between the second calibration liquid bag 80 and the calibration liquid channel 140.

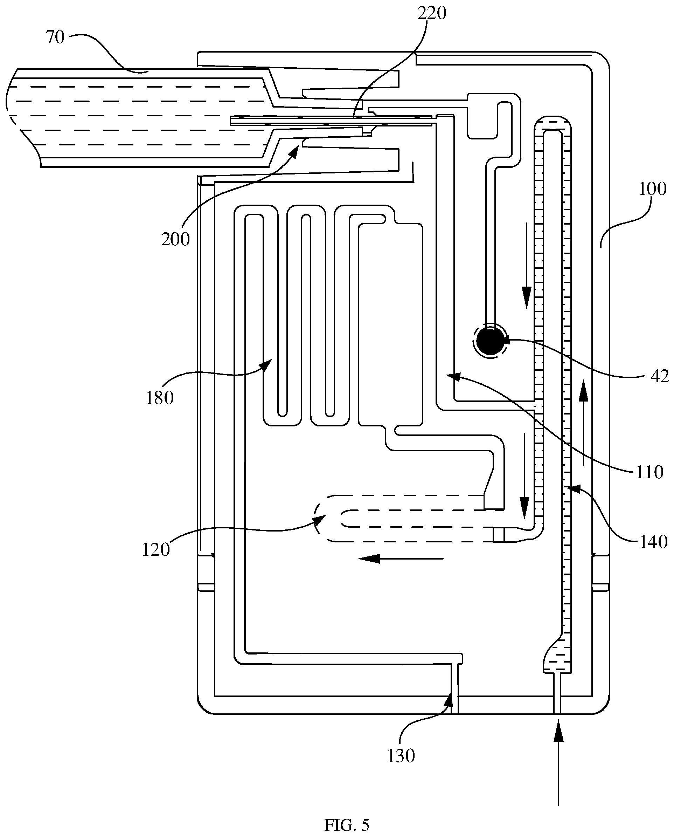

Exemplarily, as shown in FIG. 5 to FIG. 9, the controller 60 controls the action of the first retractor 40, such that the sealing end 42 is in sealing fit with the air inlet hole 232, that is, the air inlet hole 232 is closed. Then, the calibration analysis is performed. The on-off valve is opened, such that the calibration liquid in the second calibration liquid bag 80 flows into the calibration liquid channel 140. The controller 60 then controls the action of the negative pressure generator 20 to send an appropriate amount of calibration liquid into the detection chamber 120 for calibration, and uses the detection device to analyze the calibration liquid to complete the calibration analysis. The on-off valve is closed to prevent the liquid in the second calibration liquid bag 80 from interfering with subsequent detection, so as to supply liquid for the next reagent card 10. After the calibration analysis is completed, the negative pressure generator 20 continues to operate, such that the calibration liquid is extracted out of the detection chamber 120. At this time, the action of the first retractor 40 is controlled, such that the air inlet hole 232 is opened. At this time, the negative pressure generator 20 continues to operate, and an appropriate amount of sample solution (such as blood) can be sucked into the detection chamber 120. Then the detection device is used to detect the detection solution to obtain detection results of the sample solution.

Optionally, the on-off valve is an electromagnetic on-off valve, which telecommunicates with the controller 60.

In addition, the on-off valve includes, but is not limited to, a rotary valve or a linear slide valve.

On the basis of the foregoing two embodiments, as shown in FIG. 3 or FIG. 11, in an embodiment, the reagent card body 100 further includes a waste liquid storage chamber 180 communicating with a liquid outlet end of the detection chamber 120, and a liquid outlet end of the waste liquid storage chamber 180 communicates with the air receiving end 130. In this way, the calibration liquid after calibration analysis can be pushed into or sucked into the waste liquid storage chamber 180, which can not only avoid exposure of the calibration liquid, but also prevent the retention of the calibration liquid from affecting the analysis results of the detection solution. In addition, the sample solution can also be stored to avoid exposure of the sample to be tested (such as blood outflow and contamination of the instrument).

Optionally, the "detection chamber 120" is formed by, including but not limited to, a tubing or a cavity. Similarly, the "waste liquid storage chamber 180" is formed by, including but not limited to, a tubing or a cavity.

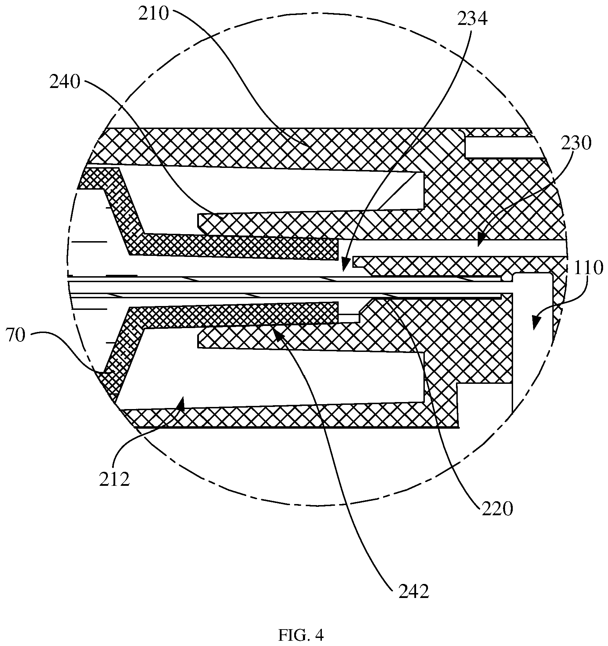

On the basis of any of the above embodiments, as shown in FIG. 4 and FIG. 9, in an embodiment, the sealing portion 240 is provided with a sealing hole 242 in sealing fit with a sample outlet end of the sample tube 70. One end of the hollow needle 220 is arranged in the sealing hole 242. The air outlet hole 234 communicates with the sealing hole 242. In this way, the inner side wall of the sealing hole 242 is closely attached to the outer side wall of the sample outlet end of the sample tube 70, such that the sample tube 70 can be hermetically and reliably installed on the installation body 210, so as to avoid air leakage and affect the detection results. In addition, the air outlet hole 234 is provided in the sealing hole 242, such that the air inlet channel 230 can provide gas for the sample tube 70 more excellently.

On the basis of any of the above embodiments, as shown in FIG. 7 and FIG. 10, in an embodiment, an outer edge of the air inlet hole 232 is provided with a sealing layer 250. In this way, the sealing layer 250 can be matched with the sealing end 42 more excellently, and the sealing effect is more excellent.

Exemplarily, as shown in FIG. 7, in an embodiment, the sealing layer 250 includes an elastic layer 252 and an adhesive layer 254 arranged on the elastic layer, and the elastic layer 252 is arranged facing outward. In this way, the elastic layer 252 can be adhered and fixed to the outer surface of the installation body 210 by the adhesive layer 254, which is easy to implement.

On the basis of any of the above embodiments, as shown in FIG. 7 or FIG. 10, in an embodiment, the sealing end 42 is provided with a protruding sealing ring 44, and the sealing ring 44 can be hermetically arranged at an outer edge of the air inlet hole 232. In this way, the air inlet hole 232 can be sealed by the sealing ring 44 more excellently. Through cooperation with the elastic layer 525 of the previous embodiment, it is beneficial to improve the sealing effect.

Optionally, the sealing ring is made of an elastic material.

Optionally, connection between "installation body 210 and reagent card body 100" includes, but is not limited to, detachable connection, such as sleeve connection and plug connection; or non-detachable connection, such as heat welding and adhesive bonding.

In the present embodiment, the installation body 210 and the reagent card body 100 are integrally formed.

Optionally, the "installation structure 200" is arranged on, including but not limited to, the reagent card 10, and may also be arranged on other structures.

The technical characteristics of the above embodiments can be arbitrarily combined. To simplify the description, all possible combinations of all the technical characteristics of the above embodiments may not be described; however, these combinations of the technical characteristics should be construed as falling within the scope defined by the specification as long as no contradiction occurs.

## Claims

1. An installation structure, comprising an installation body, wherein the installation body comprises an installation hole configured to sleeve a sample tube, a hollow needle provided in the installation hole, a sealing portion provided in the installation hole, and an air inlet channel, one end of the hollow needle is capable of being inserted into the sample tube, the sealing portion is in sealing fit with an outer wall of the sample tube, the air inlet channel comprises an air outlet hole and an air inlet hole, the air inlet hole is provided on a surface of the installation body, and the air outlet hole is configured for communication with the sample tube provided on the installation hole.

2. The installation structure according to claim 1, wherein the sealing portion is provided with a sealing hole in sealing fit with a sample outlet end of the sample tube, one end of the hollow needle is arranged in the sealing hole, and the air outlet hole communicates with the sealing hole.

3. The installation structure according to claim 1 or 2, wherein an outer edge of the air inlet hole is provided with a sealing layer.

4. The installation structure according to claim 3, wherein the sealing layer comprises an elastic layer and an adhesive layer arranged on the elastic layer, and the elastic layer is arranged facing outward.

5. A reagent card, comprising the installation structure according to any one of claims 1 to 4, and further comprising a reagent card body, wherein the reagent card body is fixed to the installation structure, the reagent card body comprises a sample inlet channel communicating with a liquid outlet end of the hollow needle, a detection chamber, and an air receiving end, and the sample inlet channel and the air receiving end communicate with the detection chamber.

6. The reagent card according to claim 5, wherein the installation body and the reagent card body are integrally formed.

7. The reagent card according to claim 5 or 6, wherein the reagent card body further comprises a calibration liquid channel, and the calibration liquid channel communicates with the detection chamber.

8. The reagent card according to claim 7, wherein the reagent card body further comprises a waste liquid storage chamber communicating with a liquid outlet end of the detection chamber, and a liquid outlet end of the waste liquid storage chamber communicates with the air receiving end.

9. The reagent card according to claim 7, wherein the reagent card body further comprises a first calibration liquid bag and a valve core, the first calibration liquid bag is fixed on the reagent card body, the first calibration liquid bag is provided with a matching portion in connection with the calibration liquid channel, the valve core is arranged in the calibration liquid channel or in the first calibration liquid bag, and the valve core is provided with a spiked portion configured to pierce the matching portion.

10. An *in-vitro* diagnostic analyzer, comprising the reagent card according to any one of claims 5 to 8, and further comprising a negative pressure generator, a sample tube, a first retractor, a detection device, and a controller, wherein the negative pressure generator is in connection with the air receiving end, the sample tube is provided with a sample outlet end in clearance fit with the hollow needle, the first retractor is provided with a sealing end configured to seal the air inlet hole, the controller telecommunicates with the negative pressure generator, the first retractor, and the detection device, and a detection end of the detection device is arranged in the detection chamber.

11. The *in-vitro* diagnostic analyzer according to claim 10, wherein the sealing end is provided with a protruding sealing ring, and the sealing ring can be hermetically arranged at an outer edge of the air inlet hole.

12. The *in-vitro* diagnostic analyzer according to claim 10 or 11, wherein the reagent card body further comprises a calibration liquid channel communicating with the detection chamber, a first calibration liquid bag, and a valve core, the first calibration liquid bag is fixed on the reagent card body, the first calibration liquid bag is provided with the matching portion in connection with the calibration liquid channel, the valve core is arranged in the calibration liquid channel or in the first calibration liquid bag, and the valve core is provided with a spiked portion configured to pierce the matching portion; and the *in-vitro* diagnostic analyzer further comprises a second retractor that telecommunicates with the controller, and a telescopic end of the second retractor is capable of pressing the calibration liquid channel or the first calibration liquid bag in a preset direction, such that the spiked portion pierces the matching portion.

13. The *in-vitro* diagnostic analyzer according to claim 10 or 11, further comprising a calibration liquid channel communicating with the detection chamber, and a second calibration liquid bag, wherein the second calibration liquid bag communicates with the calibration liquid channel through an on-off valve.
